# EUROPEAN PATENT SPECIFICATION

(11) **EP 2 246 434 B1**
(45) Date of publication and mention of the grant of the patent: **27.03.2013**
(21) Application number: 09171789.2
(22) Date of filing: 30.09.2009
(51) Int. Cl.: C12N 15/85, A01K 67/027

(54) **A GNMT- knockout mouse model for depression, schiozophrenia and Alzheimer's disease**
GNMT- knockout Mausmodell für Depression, Schizophrenie und Alzheimerkrankheit
Souris knockout pour GNMT comme modèle de dépression, schizophrénie et maladie d'Alzheimer

(30) Priority: 28.04.2009 US 431641
(43) Date of publication of application: 03.11.2010
(73) Proprietor: National Yang-Ming University, Taipei 112 (TW)
(72) Inventor: Chen, Yi-Ming, National Yang-Ming University, Taipei 112 (TW); Yang, Ching-Ping, c/o National Yang-Ming University, Taipei 112 (TW)
(74) Representative: Viering, Jentschura & Partner

(56) References cited:
- EP-A2- 2 020 443
- ZIGMUND LUKA ET AL: "A Glycine N-methyltransferase Knockout Mouse Model for Humans with Deficiency of this Enzyme" TRANSGENIC RESEARCH, KLUWER ACADEMIC PUBLISHERS-PLENUM PUBLISHERS, NE LNKD- DOI:10.1007/S11248-006-0008-1, vol. 15, no. 3, 1 June 2006 (2006-06-01), pages 393-397, XP019409553 ISSN: 1573-9368
- MARTINEZ-CHANTAR M L ET AL: "Loss of the Glycine N-Methyltransferase gene leads to steatosis and hepatocellular carcinoma in mice" HEPATOLOGY, WILLIAMS AND WILKINS, BALTIMORE, MD, US LNKD- DOI:10.1002/HEP.22159, vol. 47, no. 4, 1 April 2008 (2008-04-01), pages 1191-1199, XP002545802 ISSN: 0270-9139 [retrieved on 2007-12-19]
- LIU S-P ET AL: "Glycine N-methyltransferase-/- mice develop chronic hepatitis and glycogen storage disease in the liver" HEPATOLOGY, WILLIAMS AND WILKINS, BALTIMORE, MD, US LNKD- DOI:10.1002/HEP.21863, vol. 46, no. 5, 1 November 2007 (2007-11-01), pages 1413-1425, XP002545801 ISSN: 0270-9139 [retrieved on 2007-10-15]
- MARTINEZ-LOPEZ N ET AL: "S-adenosylmethionine and proliferation: new pathways, new targets" BIOCHEMICAL SOCIETY TRANSACTIONS, PORTLAND PRESS LTD, GB LNKD- DOI:10.1042/BST0360848, vol. 36, no. 5, 1 January 2008 (2008-01-01), pages 848-852, XP002545803 ISSN: 0300-5127
- YEN C H ET AL: "Glycine N-methyltransferase affects the metabolism of aflatoxin B1 and blocks its carcinogenic effect" TOXICOLOGY AND APPLIED PHARMACOLOGY, ACADEMIC PRESS, US LNKD- DOI:10.1016/J.TAAP.2008.12.013, vol. 235, no. 3, 15 March 2009 (2009-03-15), pages 296-304, XP025991897 ISSN: 0041-008X [retrieved on 2009-02-26]
- YI-JEN LIAO ET AL: "Characterization of a glycine N-methyltransferase gene knockout mouse model for hepatocellular carcinoma: Implications of the gender disparity in liver cancer susceptibility" INTERNATIONAL JOURNAL OF CANCER, JOHN WILEY & SONS, INC, UNITED STATES, SWITZERLAND, GERMANY LNKD- DOI:10.1002/IJC.23979, vol. 124, no. 4, 15 February 2009 (2009-02-15), pages 816-826, XP002545804 ISSN: 0020-7136 [retrieved on 2008-09-09]
- CHARLES DUYCKAERTS ET AL: "Alzheimer disease models and human neuropathology: similarities and differences" ACTA NEUROPATHOLOGICA, SPRINGER, BERLIN, DE LNKD- DOI:10.1007/S00401-007-0312-8, vol. 115, no. 1, 16 November 2007 (2007-11-16), pages 5-38, XP019562927 ISSN: 1432-0533
- ULREY CL, LIU L, ANDREWS LG, TOLLEFSBOL TO.: "The impact of metabolism on DNA methylation." HUM MOL GENET., vol. 14, no. 1, 15 April 2005 (2005-04-15) , pages R139-R147, XP002589094
- BARIC I.: "Inherited disorders in the conversion of methionine to homocysteine." J INHERIT METAB DIS., vol. 32, no. 4, 7 July 2009 (2009-07-07), pages 459-471, XP002589095
- VARELA-REY M ET AL: "Impaired Liver Regeneration in Mice Lacking Glycine N-Methyltransferase" HEPATOLOGY, WILLIAMS AND WILKINS, BALTIMORE, MD, US LNKD- DOI:10.1002/HEP.23033, vol. 50, no. 2, 1 August 2009 (2009-08-01) , pages 443-452, XP002545805 ISSN: 0270-9139 [retrieved on 2009-04-17]
- WAGNER C ET AL: "Hepatocellular carcinoma in GNMT-/- mice" TOXICOLOGY AND APPLIED PHARMACOLOGY, ACADEMIC PRESS, US LNKD- DOI:10.1016/J.TAAP.2009.03.017, vol. 237, no. 2, 1 June 2009 (2009-06-01), page 246, XP026103954 ISSN: 0041-008X [retrieved on 2009-04-01]

## Description

### Field of the invention

The present invention relates to Glycine N-methyltransferase (GNMT) knockout animal model and use thereof.

### Background of the invention

Glycine N-methyltransferase (GNMT), also known as a 4S polycyclic aromatic hydrocarbon (PAH) binding protein, has multiple functions. In addition to acting as a major folate binding protein (Yeo EJ, et al. Proc Natl Acad Sci U S A 1994;91:210-214), it also regulates the ratio of S-adenosylmethionine (SAM) to S-adenosylhomocysteine (SAH) by catalyzing sarcosine synthesized from glycine (Kerr SJ. J Biol Chem 1972;247:4248-4252). It was previously reported that the GNMT gene is down-regulated in HCC (Liu HH, et al. J Biomed Sci 2003;10:87-97). Results from a genetic epidemiological study indicate that GNMT is a tumor susceptibility gene for liver cancer (Tseng TL, et al. Cancer Res 2003;63:647-654). In addition, it was reported that GNMT binds benzo(a)pyrene and prevents DNA-adduct formation (Chen SY, et al. Cancer Res 2004;64:3617-3623).

In mice, GNMT expression is regulated by growth hormone, with the hepatocytes of female mice having up to eight times the expression level normally found in male mice. There have been three reports of pediatric patients (two boys, one girl) with congenital GNMT deficiencies resulting from a missense mutation in the GNMT gene (Augoustides-Savvopoulou P, et al. J Inherit Metab Dis 2003;26:745-759). All three children had hypermethioninaemia, clinical symptoms mimicking chronic hepatitis (Augoustides-Savvopoulou P, et al. J Inherit Metab Dis 2003;26:745-759). The girl had stunted growth and suffered from mental deficiency (IQ 87).

The prior art disclosed a GNMT knock-out mouse which showed abnormal liver function and suffered from glycogen storage disease (US Application No. 11/832,304). DUYCKAERTS et al, ACTA NEUROPATHOLOGICA, 2007 115 (1) p 5-38, reviews Alzheimer disease models and human neuropathology but does not disclose any relation with GNMT.

### Summary of the invention

The present invention provides a knockout mouse model for studying depression, schizophrenia or Alzheimer's disease, wherein the Knockout model is a GNNTY mouse whose genome is homozygously disrupted by recombination at Glycine N-methyltransferase (GNMT) gene locus.

The present invention also provides a method of generating an mouse exhibiting a pathological condition of depression, schizophrenia or Alzheimer's disease, comprising homozygous disruption of GNMT gene in the animal by recombination at GNMT gene locus.

The present invention further provides a method for screening a drug candidate for treating depression, schizophrenia or Alzheimer's disease in a subject, comprising: (a) administering a potential drug candidate to the GNMT-/-mouse model of claim 1, (b) measuring the response of said animal to said drug candidate, (c) comparing the response of said animal with that of an animal having a wild type GNMT gene, and (d) selecting the drug candidate based on the difference in response observed between said animal and said animal having a wild type GNMT gene.

### Brief description of the drawings

Fig. 1 shows the strategy of constructing the targeting vector.

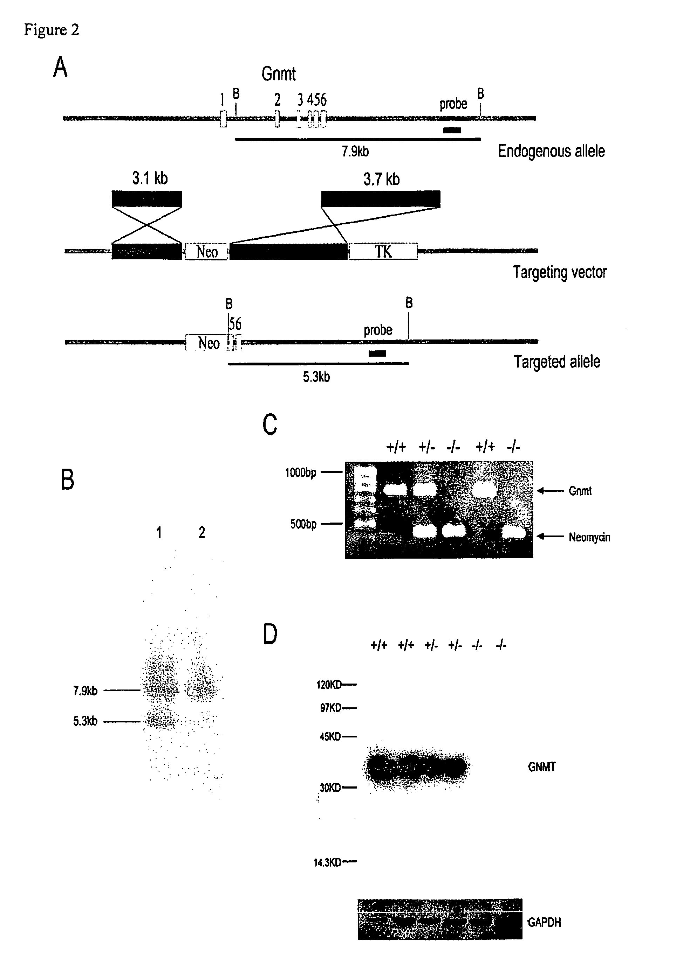

Fig. 2 shows targeted modification of the GNMT gene locus. (A) Targeting vector was designed to replace GNMT exons 1-4 and a part of exon 5 with a neomycin resistance gene. *Neomycin* positive selection marker is flanked by two homologous regions and followed by a *TK* negative selection marker at the 3' end of the targeting vector. (B) Southern blot analysis of embryonic stem cell clones. BamHI (B)-BamHI DNA fragment size decreased from 7.9 kb (wild-type allele) to 5.3 kb (recombinant allele). (C) Genotyping of GNMT knockout mice by PCR. The normal GNMT allele yielded a 772 bp fragment and the disrupted allele a 409bp fragment. +/+, wild-type; +/-, GNMT heterozygous and -/-, GNMT-/- mice (D) Expression of GNMT protein confirmed by western blot analysis. Each lane contains 10 µg hepatic lysate. GNMT molecular mass: 32 kDa. GAPDH: internal control.
Fig. 3 shows GNMT-/- male mice displayed significant deficits in prepulse inhibition of the acoustic startle reflex. Data are presented as mean± S.E.M.; ** *p* < 0.01.
Fig. 4 shows using TST and FST, GNMT-/- displayed significant increased immobility in the TST (A) and FST (B). Data are presented as mean± S.E.M.; * *p* < 0.05.
Fig. 5 shows that no significant difference of locomotor activity was found between both sexes of WT and GNMT-/- mice.
Fig. 6 shows GNMT-/- mice having motor deficits of shorter latency of falling from the Rotating rod task. Data are presented as mean± S.E.M.; * *p* < 0.05.
Fig. 7 shows comparing the following Alzheimer's Disease associated mRNA expression in the cerebral cortex of one-month old mice by Q-PCR, as follows, APP, BACE 1, BACE 2, APH-1α, GSK-3β, MAPT, SNCα and IDE. Data are presented as mean± S.E.M.; * *p* < 0.05.
Fig. 8 shows that together with nestin (A), GNMT (B) expression was found on neural progenitor cell in immunofluoresencent images.
Fig. 9 shows detecting of GNMT expression in WT mouse brain (a) GNMT mRNA in different parts of mouse brain using RT-PCR. 1) olfactory bulb, 2) cortex, 3) striatum, 4) midbrain, 5) cerebellum, 6) spinal cord, 7) hippocampus, 8) thalamus and hypothalamus, 9) pons and medulla, 10) brain stem. (b) Immunostaining of GNMT in WT mice brain.

### Detailed description of the invention

The GNMT gene expresses in the neural progenitor cell and partial region of the brain, such as cortex, straitum and substantia nigra. Besides, the Depression-like and Schizo-like behaviors were observed in the Gnmt knock-out mouse model. Furthermore, the GNMT gene expresses in the mouse neuron cells which develops *in vitro*. Those results indicate the GNMT gene plays essential role in the function of brain and development.

According to the experimental results, it shows that metabolite of dopamine, dihydroxyphenylacetic acid, of the GNMT-/- knock-out mouse is apparently reduced. Besides, it is observed that the GNMT-/- ock-out mice display significant deficits in prepulse inhibition of the acoustic startle test compared with wild-type mouse. It is also observed that GNMT-/-knock-out mouse is easier to give up to the TST (tail suspension test) and FST (force swim test). From the RotaRod motor test, the result shows the exercise ability of GNMT knock-out mouse is inferior to that of wild-type mouse. The GNMT-/- knock-out mouse presents Depression-like and Schizo-like behavior. Furthermore, the results of microarray show GNMT deficiency results in the increase expression of Alzheimer's disease related genes. Hence, the present mouse model could be applied to research the Depression, Schizophrenia and Alzheimer's disease.

The present invention also provides a method of generating a mouse exhibiting a pathological condition of depression, schizophrenia or Alzheimer's disease, comprising homozygous disruption of GNMT gene in the mouse by recombination at GNMT gene locus. The pathological condition is characterized by deficits in prepulse inhibition of acoustic startle reflex, decreased immobility of tail suspension test and forced swim test, or elevating expression of Alzheimer's disease-associated genes. The elevating expression of Alzheimer's disease-associated genes includes but is not limited to BACE 1, BACE 2, APH-1, GSK-3, MAPT, and IDE.

The present invention further provides a method for screening a drug candidate for preventing or treating depression, schizophrenia or Alzheimer's disease in a subject, comprising: (a) administering a potential drug candidate to the GNMT-/- mouse model of the present invention, (b) measuring the response of said GNMT-/- mouse to said drug candidate, (c) comparing the response of said GNMT-/- mouse with that of mouse having a wild type GNMT gene, and (d) selecting the drug candidate based on the difference in response observed between said GNMT-/- mouse and said mouse having a wild type GNMT gene.

As used herein, "drug candidate" means a composition of matter that is being investigated for a pharmacological or other activity or that is known to have a pharmacological or other activity, but is being tested to see if it has any type of activity in a particular subject, such as a patient. The drug candidate includes but is not limited to nucleic acid, peptide, and chemical compound. Efficacy of a drug candidate is one example of a pharmacological activity. Moreover, clinical outcome can be characterized as an activity of a drug candidate.

The present disclosure also provide a method for screening a drug candidate for treating depressing, schizophrenia or Alzheimer's disease in a subject, comprising: (a) providing a GNMT-/- mouse cell comprising a disruption in an endogenous GNMT gene, wherein the disruption results in a reduced level of an GNMT biological activity in the mammalian cell as compared to that of a wild type cell under identical conditions, (b) administering the potential drug candidate to the cell of step (a), and (c) comparing the response of said cell with that of a cell having a wild type GNMT gene, and (d) selecting the drug candidate based on the difference in response observed between said cell and said cell having a wild type GNMT gene. The method of the present invention, wherein the mouse cell is present within a knockout GNMT-/- mouse, and the preferable cell is neural progenitor cell of brain.

### Examples

### Example 1: Preparing the GNMT-/- knock-out mouse

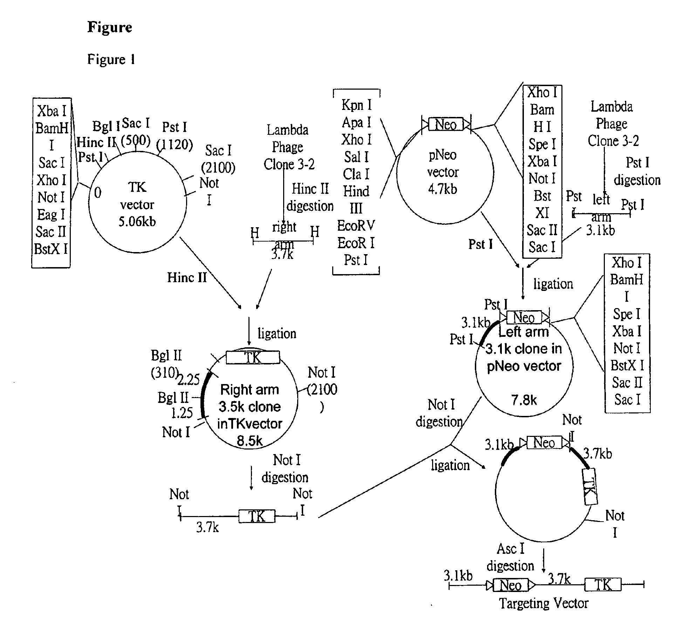

To construct a targeting vector, DNA fragments digested from lambda phage clones 3-2 and 5-3 were inserted into a plasmid-pBluescrip II KS. Left arm was digested from the phage clone 5-3 by using Pst I and inserted into the pNeo vector. Right arm was digested from the phage clone 3-2 by using Hinc II and inserted into the TK vector. The fragment containing right arm and TK gene was digested by using Not I and inserted into the pNeo vector containing left arm to generate the targeting vector (Fig. 1).

The neomycin gene (to replace exons 1-4 and part of exon 5 of the mouse Gnmt gene) was framed with two DNA fragments (3.1 kb and 3.7 kb) in the targeting vector. The thymidine kinase gene was used as a negative selection marker (Fig. 2A). The 40 µg targeting vector was linearized using AscI and introduced into embryonic stem cells (129/Sv-derived) by electroporation. After screening 278 clones using southern blot analysis (Fig. 2B), a recombinant clone was isolated and used for microinjection into blastocytes. Four male chimeric mice were obtained and used to breed female C57BL/6 mice. Agouti F₁ offspring were subjected to PCR to detect the germline transmission of the disrupted allele. Heterozygous F₁ male mice were backcross with female wild-type C57B/6 mice to generate C57BL/6 genome background mice.

PCR was developed to differentiate wild-type (+/+), GNMT heterozygous (+/-), and GNMT-/- mice. The primers used for PCR were shown as the following: GNMT-F (5'-GCGGCGGCCGCATGCTGGTGGAAGAGGGC) and GNMT-R (5'-TTGCAGTCTGGCAAGTGAGC) for GNMT; neomycin-F (5'-GTTCCTTGCGCAGCTGTGCT) and neomycin-R (5'-CGGCCACAGTCGATGAATCC) for neomycin. The normal GNMT allele yielded a 772bp fragment by GNMT primers and the disrupted allele yielded a 409bp fragment by neomycin primers (Fig. 2C). The expression of GNMT protein in liver was analyzed using western blot; the results show that compared to the wild-type, GNMT expression decreased approximately 50% in the livers of GNMT+/- mice and GNMT was undetectable in the livers of GNMT-/- mice (Fig. 2D).

### Example 2: Prepulse Inhibition (PPI) of Startle Reflex

The apparatus consisted of two startle chambers (Med Associates, Georgia, VT). One mouse selected from the WT control group and the other selected from GNMT deficient group were tested simultaneously. Each mouse was put into the PPI chamber for a 5-min acclimatization period with a 60 dB background noise. Following this period, 10 startle pulses (120 dB, 40 ms duration) were presented with an average inter-trial interval of 15 s. Then, no stimulus (background noise, 68 dB), prepulses alone (72, 76 and 84 dB, 20 ms duration), startle pulses alone, and prepulses followed 80 ms later by startle pulses were presented six times randomly distributed over the next 20 min.

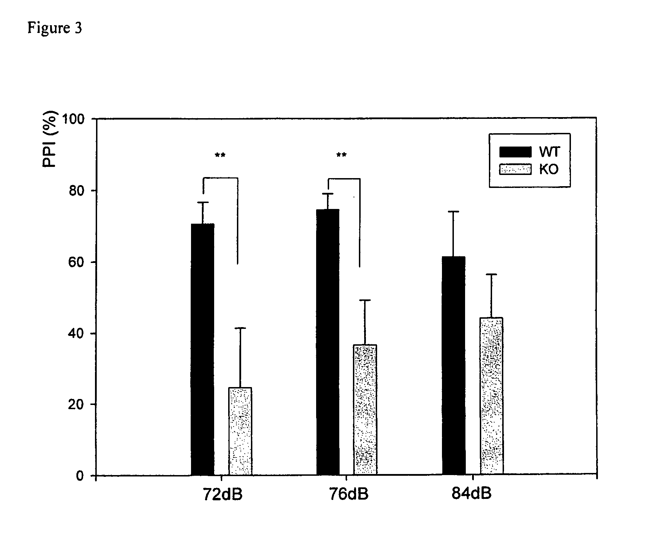

PPI was defined as the percentage reduction of startle magnitude in the presence of the prepulse compared to the magnitude in the absence of the prepulse. %PPI = [1 - (prepulse trials/startle-only trials)]×100. Figure 3 showed GNMT^{-/-} mice displayed significant deficits in prepulse inhibition of the acoustic startle reflex.

### Example 3: Tail suspension test

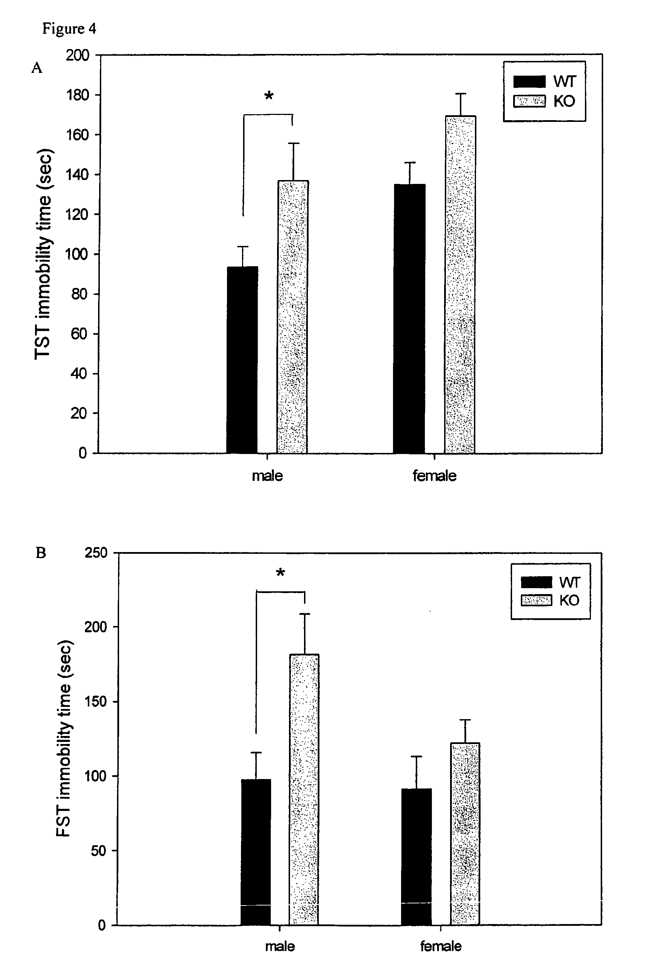

8-12 weeks old male and female mice were suspended by the tail. After 'agitation' or 'escape-like' behavior, mice adopted an immobile posture, suggested to mirror a state of depression. The immobility time during a 5 min test recorded. The result of Fig. 4A showed that GNMT^{-/-} mice displayed significant decreased immobility.

### Example 4: Forced swim test

8-12 weeks old male and female mice were placed ( n = 11 per WT and n = 13 per KO) individually in rectangular cage (height 30 cm, diameter 15 cm) filled with 12-cm-deep water (temperature 22 ± 1°C) for 6 min. The processes of the total period of immobility during the last 5 min were recorded. The immobility of GNMT^{-/-} mice was decrease in the Fig. 4B. Immobility was defined as the absence of initiated movements and includes passive swaying.

### Example 5: Analysis of Motor activity

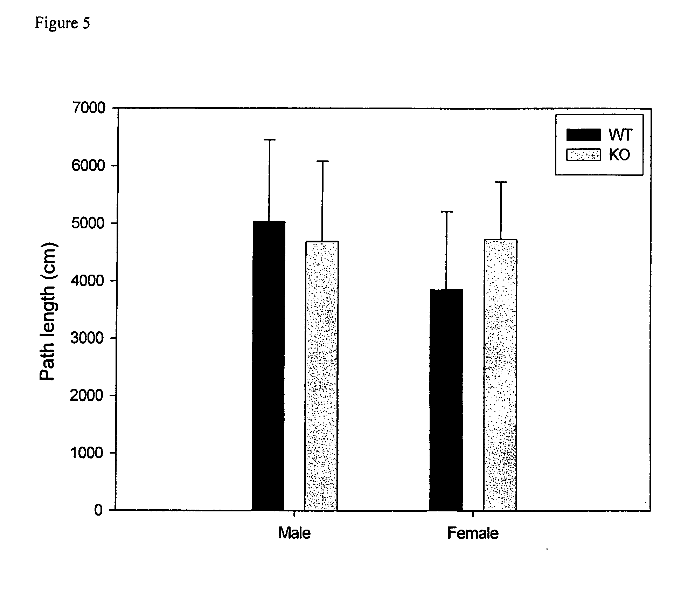

GNMT^{-/-} animal and their wild-type were individually tested for motor activity at 8-12 weeks of age under 90cm×90cm×30cm open field. Each mouse was tested for 10 min between 1700 and 1900 h. The results shown in Fig 5 were generated online by the TrackMot software package. There was no significant difference found between both sexes of WT and GNMT^{-/-} mice.

### Example 6: Rotarod testing

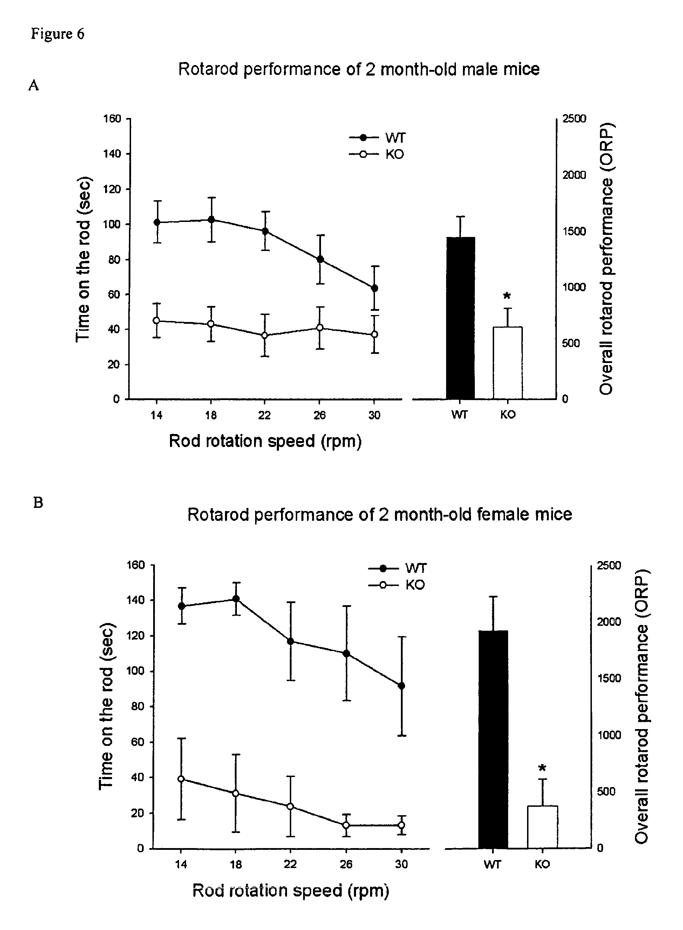

On the day of testing, all animals were transferred to the test room at least half an hour earlier. Then the mice were tested on a rotarod apparatus which consisted of a rotating rod (diameter, 3 cm; hard non-slipping plastic). All mice were habituated to the apparatus for at least four consecutive trials in which the rod was kept at constant speed (one trial at 0 rpm and three trials at 5 rpm) with 5 minutes interval. Once the trained animals were able to stay on the rod rotating at 5 rpm for 60 seconds in three consecutive trials, they proceeded to the test. Three trials at each of five fixed rotating speeds (14, 18, 22, 26, and 30 rpm) were sequentially conducted for a maximum of 150 seconds each speed or until the animals fell off. The length of time that each animal was able to stay on the rod at each rotation speed was recorded (latency to fall). Regardless of completion or fall, each animal was allowed to rest for at least 5 minutes between individual testing speeds and 30 minutes between each complete trial. The mean of overall rod performance (ORP) for the three trials of each mouse was calculated by the trapezoidal method as the area under the curve in the plot of latent time on the rod versus rotation speed. (Fig. 6)

### Example 7: RNA isolation and RT-PCR

Total RNA was extracted from tissues using TRIzol (Invitrogen, Carlsbad, CA). Complementary DNA was produced from olfactory bulb, cortex, striatum, midbrain, cerebellum, spinal cord, hippocampus, hypothalamus, medulla and brain stem RNA (5 µg) using a SuperScript II Reverse Transcriptase Kit (Invitrogen). PCR conditions were pre-denaturated at 94°C for 5 minutes followed by 30 cycles of amplification at 94°C for 30 seconds, 60°C for 30 seconds, and 72°C for 1 min, followed by a 10-minute extension step at 72°C. The primer sequences are followings: m-GNMT-F (5'-GCGGCGGCCGCATGCTGGTGGAAGAGGGC) and m-GNMT-R (5'-TTG CAGTCTGGCAAGTGAGC) for GNMT; β-actin-F (5'-GGGCGCCCCAGGCACCA) and β-actin-R (5'-CTCCTTAATGTCACGCCGATTTC) for β-actin.

### Example 8: Real-Time PCR

Ten genes belonging to the Alzheimer Disease pathway were selected for real-time PCR analysis. Real-time PCR primers were designed using PRIMER EXPRESS software (Version 2.0, Applied Biosystems) and verified the specificity of sequences using BLAST. Reactions were performed in 10µl quantities of diluted cDNA sample, primers (100 nM), and a SYBR Green PCR Master Mix containing nucleotides. Reactions were assayed using an Applied Biosystems Prism 7000 sequence detection system.

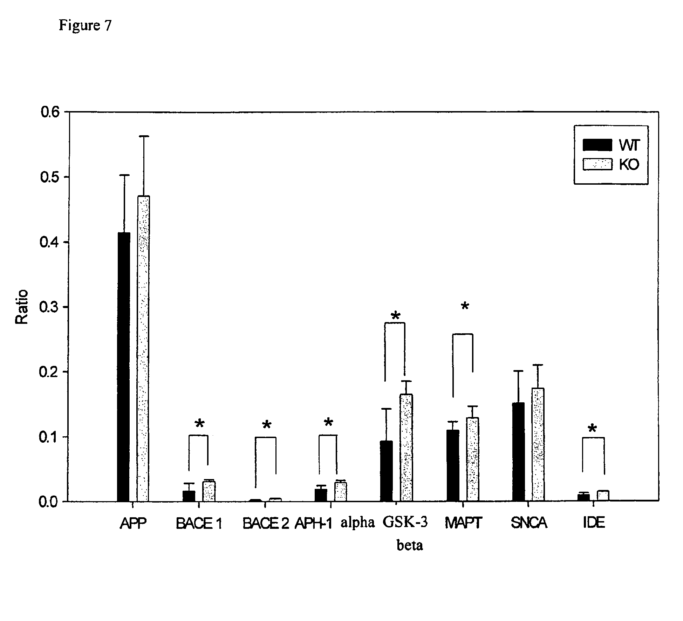

After cycling, a melting curve was produced via the slow denaturation of PCR end products to validate amplification specificity. Predicted cycle threshold (*C*_{T}) values were exported into EXCEL worksheets for analysis. Comparative *C*_{T} methods were used to determine relative gene expression folds to GAPDH. The primers used for real-time PCR were shown as the followings: APP-F (5'-GCCAGCCAATACCGAAAATG) and APP-R (5'-GATGTTTGTCAGCCCAGAACCT) for APP; BACE1-F (5'-ACGACTCTTTGGAGCCCTTCT) and BACE1-R (5'-AGAGCTGCAGGGAAAAGATGTT) for BACE1 for BACE; BACE2-F (5'-CACGGAAGACATAGCCAGCAA) and BACE2-R (5'-TCAGGGCATAGGACACAATCC) for BACE2; IDE-F (5'-CGTCCAATCTGATGGCGATT) and IDE-R (5'-AGAACAGCTTCACCACCAGGTTA) for IDE; SNCA-F (5'-AAACACCTAAGTGACTACCACTTATTTCTAAA) and SNCA-R (5'-TCTTGGAGCAAATCACAACTTCTT) for SNCA; MAPT-F (5'-AGCAATGAGAGATTTGAGACTTGGT) and MAPT-R (5'-CCTTCGCTGTCGCTGTTTC) for MAPT; APH1α-F (5'-ATGCACGGCTCCAGTATGG) and APH1α-R (5'-GCAAAACGGAACACTTCCTGTAG) for APH1α; GSK3β-F (5'-CGGGACCCAAATGTCAAACT) and GSK3β-R (5'-TCCGAGCATGTGGAGGGATA) for GSK3β; GAPDH-F (5'-TGGTATCGTGGAAGGACTCA) and GAPDH-R (5'-AGTGGGTGTCGCTGTTGAAG) for GAPDH. (Fig. 7)

### Example 9: Immunofluorescent staining

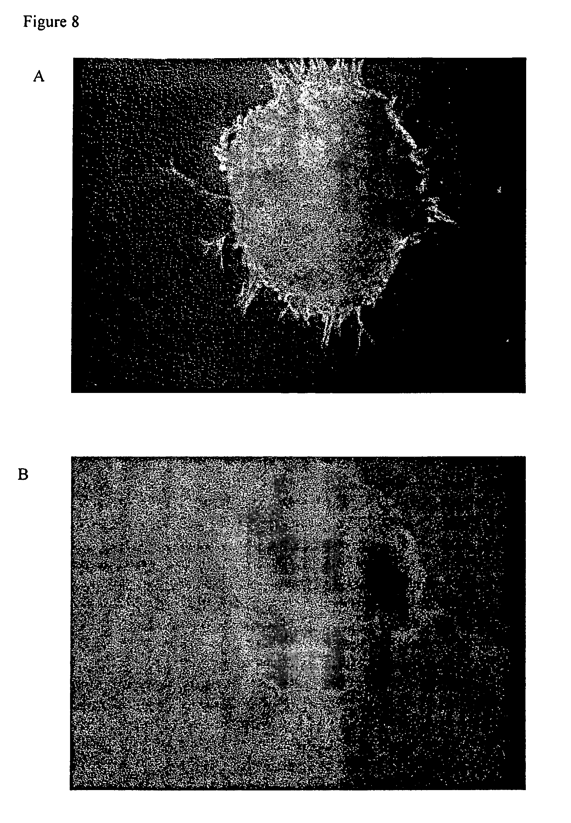

Neural progenitor cell culture followed the protocol by Zhou. et al. After 7 or 10 days in the culture of subplating, the subcultures were washed with cold 0.1 M PBS three times and then fixed with 4% paraformaldehyde for 4 h and permeated with 0.1% Triton X-100 for 30 min. The phenotypic expression of the neurospheres was examined using immunocytochemical staining with antibodies against (a) Nestin (1:500) (BD Biosciences) for neuroepithelial stem cells or (b) GNMT (1:250). (Fig. 8)

### Example 10: Immunohistochemistry

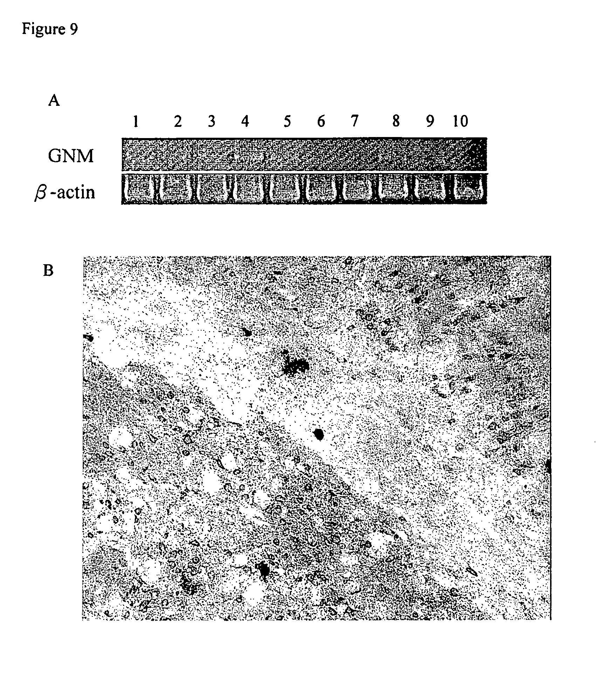

Isolated brain tissues of WT mice are fixed in 10% neutral-buffered formalin. After infiltrating with 30% sucrose solution in PBS, cut the tissue using frozen sections and paraffin sections (method). For antigen retrieval, tissue sections on slides were immersed in borate buffer solution (pH 8) jar and placed in pressure oven for about 20 min until the cooker reached its maximum pressure. It was then heated for another 5 min at maximum pressure. Thereafter, the pressure was reduced and cooled in a bath of tap water. Then the sections incubate in blocking solution at room temperature for 6 hours. And they were incubated overnight at 4°C with the following rabbit anti-GNMT sera at 1/100. After washing in PBS, these slides were incubated with biotinylated antibody and peroxidase-labeled streptavidin (DAKO, Carpinteria, CA) for 10 min at room temperature. These slides were further incubated with 3,3'-diaminobenzidine tetrahydrochloride solution for color reaction. (Fig. 9)

### Example 11_{:} Statistical analysis

All data were pooled according to genotype, and a mean value was determined for each group. Results were presented as means + SEM and were analyzed by Student's t-test with p≦0.05 used as significance criteria.

One skilled in the art readily appreciates that the present invention is well adapted to carry out the objects and obtain the ends and advantages mentioned, as well as those inherent therein.

It will be readily apparent to a person skilled in the art that varying substitutions and modifications may be made to the invention disclosed herein without departing from the scope of the invention.

The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. Use of a GNMT-/- mouse whose genome is disrupted by recombination at the Glycine N-Methyltransferase (GNMT) gene locus for studying depression, schizophrenia or Alzheimer's disease.

2. A method of generating a mouse model exhibiting a pathological condition of depression, schizophrenia or Alzheimer's disease, by homozygously disruption disrupting one Glycine N-Methyltransferase (GNMT) gene in the mouse by recombination at the GNMT gene locus.

3. The method of claim 2, wherein the pathological condition is **characterized by** deficits in prepulse inhibition of acoustic startle reflex, decreased immobility of tail suspension test and forced swim test, or elevating expression of Alzheimer's disease-associated genes.

4. The method of claim 3, wherein the Alzheimer's disease-associated genes are BACE 1, BACE 2, APH-1, GSK-3, MAPT, and IDE.

5. A method for screening a drug candidate for treating depression, schizophrenia or Alzheimer's disease in a subject, comprising:
(a) administering a potential drug candidate to a GNMT-/- mouse whose genome is homozygously disrupted by recombination at Glycine N-methyltransferase (GNMT) gene locus, and exhibiting a pathological condition of depression, schizophrenia or Alzheimer's disease,
(b) measuring the response of said GNMT-/- mouse to said drug candidate,
(c) comparing the response of said GNMT-/- mouse with that of a mouse having a wild type GNMT gene, and
(d) selecting the drug candidate based on the difference in response observed between said GNMT-/- mouse and said mouse having a wild type GNMT gene.

6. The method of claim 5, wherein the response is acoustic startle reflex, tail suspension test, or forced swim test.

## Patentansprüche

1. Verwendung einer GNMT-/- Maus, deren Genom durch eine Rekombination des Glycin N-Methyltransferase (GNMT) Genlocus beeinträchtigt ist, zur Untersuchung von Depression, Schizophrenie oder der Alzheimer-Krankheit.

2. Verfahren zur Herstellung eines Mausmodels, das eine pathologische Erkrankung von Depression, Schizophrenie oder der Alzheimer-Krankheit zeigt, durch homozygote Zerstörung eines Glycin N-Methyltransferase (GNMT) Gens der Maus durch Rekombination am GNMT Genlocus.

3. Das Verfahren nach Anspruch 2, wobei die pathologische Erkrankung durch Mangel der Präpulsinhibition des akustischen Schreckreflexes, verminderte Immobilität im Schwanz-Suspension-Test und erzwungenem Schwimm-Test oder gesteigerte Expression von mit der Alzheimer-Krankheit assoziierten Genen gekennzeichnet ist.

4. Das Verfahren nach Anspruch 3, wobei die mit der Alzheimer-Krankheit assoziierten Gene BACE 1, BACE 2, APH-1, GSK-3, MAPT und IDE sind.

5. Verfahren zum Screnning eines Wirkstoffkandidaten zur Behandlung von Depression, Schizophrenie oder der Alzheimer-Krankheit in einem Subjekt, umfassend:
(a) Verabreichen eines potentiellen Wirkstoffes an eine GNMT-/- Maus, deren Genom homozygot durch eine Rekombination des Glycin N-Methyltransferase (GNMT) Genlocus zerstört ist und die eine pathologische Erkrankung von Depression, Schizophrenie oder der Alzheimer-Krankheit, zeigt,
(b) Messen des Ansprechens der GNMT-/- Maus auf den Wirkstoffkandidaten,
(c) Vergleichen des Ansprechens der GNMT-/- Maus mit dem Ansprechen einer Maus, die eine Wildtyp Variante des GNMT Gens besitzt,
(d) Auswählen des Wirkstoffkandidaten auf der Grundlage des beobachteten Unterschieds im Ansprechverhalten zwischen der GNMT-/- Maus und der Maus, die eine Wildtyp Variante des GNMT Gens besitzt.

6. Das Verfahren nach Anspruch 5, wobei das Ansprechen der akustische Schreckreflex, der Schwanz-Suspension-Test oder der erzwungene Schwimm-Test ist.

## Revendications

1. Utilisation d'une souris GNMT-/- dont le génome est interrompu par recombinaison au niveau du locus du gène de glycine N-méthyltransférase (GNMT) pour étudier la dépression, la schizophrénie ou la maladie d'Alzheimer.

2. Procédé pour générer un modèle de souris présentant un état pathologique de dépression, de schizophrénie ou de maladie d'Alzheimer, par interruption homozygote du gène de glycine N-méthyltransférase (GNMT) chez la souris par recombinaison au niveau du locus du gène de GNMT.

3. Procédé selon la revendication 2, dans lequel l'état pathologique est **caractérisé par** des déficits de l'inhibition par pré-stimulus du réflexe de sursaut acoustique, une réduction de l'immobilité lors d'un test de suspension par la queue et d'un test de nage forcée, ou une élévation de l'expression de gènes associés à la maladie d'Alzheimer.

4. Procédé selon la revendication 3, dans lequel les gènes associés à la maladie d'Alzheimer sont BACE 1, BACE 2, APH-1, GSK-3, MAPT, et IDE.

5. Procédé pour dépister un médicament candidat pour le traitement de la dépression, de la schizophrénie ou de la maladie d'Alzheimer chez un sujet, comprenant :
(a) l'administration d'un médicament candidat potentiel à une souris GNMT-/- dont le génome est interrompu de façon homozygote par recombinaison au niveau du locus du gène de glycine N-méthyltransférase (GNMT), et présentant un état pathologique de dépression, de schizophrénie ou de maladie d'Alzheimer,
(b) la mesure de la réponse de ladite souris GNMT-/- audit médicament candidat,
(c) la comparaison de la réponse de ladite souris GNMT-/- à celle d'une souris ayant un gène GNMT de type sauvage, et
(d) la sélection du médicament candidat sur la base de la différence de réponse observée entre ladite souris GNMT-/- et ladite souris ayant un gène GNMT de type sauvage.

6. Procédé selon la revendication 5, dans lequel la réponse est un réflexe de sursaut acoustique, un test de suspension par la queue, ou un test de nage forcée.


### SEQUENCE LISTING

<110> National Yang Ming university
<120> A mouse model for depression, schizophrenia and Alzheimer's disease
<130> P40100
<150> US 12/431,641
   <151> 2009-04-28
<160> 26
<170> PatentIn version 3.5
<210> 1
   <211> 29
   <212> DNA
   <213> Artificial Sequence
<220>
   <223> GNMT-F
<220>
   <221> misc_feature
   <222> (1)..(29)
<400> 1
   gcggcggccg catgctggtg gaagagggc 29
<210> 2
   <211> 20
   <212> DNA
   <213> Artificial sequence
<220>
   <223> GNMT-R
<220>
   <221> misc_feature
   <222> (1)..(20)
<400> 2
   ttgcagtctg gcaagtgagc 20
<210> 3
   <211> 20
   <212> DNA
   <213> Artificial Sequence
<220>
   <223> neomycin-F
<220>
   <221> misc_feature
   <222> (1)..(20)
<400> 3
   gttccttgcg cagctgtgct 20
<210> 4
   <211> 20
   <212> DNA
   <213> Artificial Sequence
<220>
   <223> neomycin-R
<220>
   <221> misc_feature
   <222> (1)..(20)
<400> 4
   cggccacagt cgatgaatcc 20
<210> 5
   <211> 29
   <212> DNA
   <213> Artificial sequence
<220>
   <223> m-GNMT-F
<220>
   <221> misc_feature
   <222> (1)..(29)
<400> 5
   gcggcggccg catgctggtg gaagagggc 29
<210> 6
   <211> 20
   <212> DNA
   <213> Artificial sequence
<220>
   <223> m-GNMT-R
<220>
   <221> misc_feature
   <222> (1)..(20)
<400> 6
   ttgcagtctg gcaagtgagc 20
<210> 7
   <211> 17
   <212> DNA
   <213> Artificial Sequence
<220>
   <223> beta-actin-F
<220>
   <221> misc_feature
   <222> (1)..(17)
<400> 7
   gggcgcccca ggcacca 17
<210> 8
   <211> 23
   <212> DNA
   <213> Artificial Sequence
<220>
   <223> beta-actin-R
<220>
   <221> misc_feature
   <222> (1)..(23)
<400> 8
   ctccttaatg tcacgccgat ttc 23
<210> 9
   <211> 20
   <212> DNA
   <213> Artificial Sequence
<220>
   <223> APP-F
<220>
   <221> misc_feature
   <222> (1)..(20)
<400> 9
   gccagccaat accgaaaatg 20
<210> 10
   <211> 22
   <212> DNA
   <213> Artificial Sequence
<220>
   <223> APP-R
<220>
   <221> misc_feature
   <222> (1)..(22)
<400> 10
   gatgtttgtc agcccagaac ct 22
<210> 11
   <211> 21
   <212> DNA
   <213> Artificial Sequence
<220>
   <223> BACE1-F
<220>
   <221> misc_feature
   <222> (1)..(21)
<400> 11
   acgactcttt ggagcccttc t 21
<210> 12
   <211> 22
   <212> DNA
   <213> Artificial sequence
<220>
   <223> BACE1-R
<220>
   <221> misc_feature
   <222> (1)..(22)
<400> 12
   agagctgcag ggaaaagatg tt 22
<210> 13
   <211> 21
   <212> DNA
   <213> Artificial sequence
<220>
   <223> BACE2-F
<220>
   <221> misc_feature
   <222> (1)..(21)
<400> 13
   cacggaagac atagccagca a 21
<210> 14
   <211> 21
   <212> DNA
   <213> Artificial sequence
<220>
   <223> BACE2-R
<220>
   <221> misc_feature
   <222> (1)..(21)
<400> 14
   tcagggcata ggacacaatc c 21
<210> 15
   <211> 20
   <212> DNA
   <213> Artificial sequence
<220>
   <223> IDE-F
<220>
   <221> misc_feature
   <222> (1)..(20)
<400> 15
   cgtccaatct gatggcgatt 20
<210> 16
   <211> 23
   <212> DNA
   <213> Artificial Sequence
<220>
   <223> IDE-R
<220>
   <221> misc_feature
   <222> (1)..(23)
<400> 16
   agaacagctt caccaccagg tta 23
<210> 17
   <211> 32
   <212> DNA
   <213> Artificial Sequence
<220>
   <223> SNCA-F
<220>
   <221> misc_feature
   <222> (1)..(32)
<400> 17
   aaacacctaa gtgactacca cttatttcta aa 32
<210> 18
   <211> 24
   <212> DNA
   <213> Artificial Sequence
<220>
   <223> SNCA-R
<220>
   <221> misc_feature
   <222> (1)..(24)
<400> 18
   tcttggagca aatcacaact tctt 24
<210> 19
   <211> 25
   <212> DNA
   <213> Artificial Sequence
<220>
   <223> MAPT-F
<220>
   <221> misc_feature
   <222> (1)..(25)
<400> 19
   agcaatgaga gatttgagac ttggt 25
<210> 20
   <211> 19
   <212> DNA
   <213> Artificial sequence
<220>
   <223> MAPT-R
<220>
   <221> misc_feature
   <222> (1)..(19)
<400> 20
   ccttcgctgt cgctgtttc 19
<210> 21
   <211> 19
   <212> DNA
   <213> Artificial Sequence
<220>
   <223> APH1 alpha-F
<220>
   <221> misc_feature
   <222> (1)..(19)
<400> 21
   atgcacggct ccagtatgg 19
<210> 22
   <211> 23
   <212> DNA
   <213> Artificial sequence
<220>
   <223> APH1 alpha-R
<220>
   <221> misc_feature
   <222> (1)..(23)
<400> 22
   gcaaaacgga acacttcctg tag 23
<210> 23
   <211> 20
   <212> DNA
   <213> Artificial Sequence
<220>
   <223> GSK3 beta-F
<220>
   <221> misc_feature
   <222> (1)..(20)
<400> 23
   cgggacccaa atgtcaaact 20
<210> 24
   <211> 20
   <212> DNA
   <213> Artificial Sequence
<220>
   <223> GSK3 beta-R
<220>
   <221> misc_feature
   <222> (1)..(20)
<400> 24
   tccgagcatg tggagggata 20
<210> 25
   <211> 20
   <212> DNA
   <213> Artificial sequence
<220>
   <223> GAPDH-F
<220>
   <221> misc_feature
   <222> (1)..(20)
<400> 25
   tggtatcgtg gaaggactca 20
<210> 26
   <211> 20
   <212> DNA
   <213> Artificial Sequence
<220>
   <223> GAPDH-R
<220>
   <221> misc_feature
   <222> (1)..(20)
<400> 26
   agtgggtgtc gctgttgaag 20